# EUROPEAN PATENT SPECIFICATION

(11) **EP 4 070 805 B1**
(45) Date of publication and mention of the grant of the patent: **16.04.2025**
(21) Application number: 20889353.7
(22) Date of filing: 19.11.2020
(51) Int. Cl.: A61K 35/745, A61P 1/00, A23L 33/135, A23K 10/18, A23C 9/123, A23C 19/032, A23K 10/16

(54) **COMPOSITION COMPRISING BIFIDOBACTERIUM LACTIS BL-99 FOR USE IN INHIBITING INTESTINAL INFLAMMATION**
ZUSAMMENSETZUNG ENTHALTEND BIFIDOBACTERIUM LACTIS BL-99 ZUR VERWENDUNG IN DER HEMMUNG VON INTESTINALEN ENTZÜNDUNGEN
COMPOSITION COMPRENANT DU BIFIDOBACTERIUM LACTIS BL-99 POUR UTILISATION DANS L'INHIBITION DE L'INFLAMMATION INTESTINALE

(30) Priority: 20.11.2019 CN 201911139551
(43) Date of publication of application: 12.10.2022
(73) Proprietor: Inner Mongolia Yili Industrial Group Co., Ltd., Hohhot, Inner Mongolia 010110 (CN); Inner Mongolia Dairy Tech Res Institute Co Ltd, Hohhot, Inner Mongolia 010110 (CN)
(72) Inventor: HUNG, Wei-Lian, Hohhot, Inner Mongolia 010110 (CN); ZHAO, Wen, Hohhot, Inner Mongolia 010110 (CN); LIU, Wei-Hsien, Hohhot, Inner Mongolia 010110 (CN); ZHANG, Haibin, Hohhot, Inner Mongolia 010110 (CN); YIN, Xiaojing, Hohhot, Inner Mongolia 010110 (CN)
(74) Representative: Dehns
(86) International application number: PCT/CN2020/129990
(87) International publication number: WO 2021/098755

(56) References cited:
- WO-A1-2007/140622
- WO-A1-2010/099824
- CN-A- 102 438 637
- CN-A- 110 157 650
- CN-A- 110 893 194
- CN-A- 110 964 655
- CN-A- 110 964 657
- US-A1- 2008 274 084
- CHAE JUNG MIN ET AL: "Effects of Orally-Administered Bifidobacterium animalis subsp. lactis Strain BB12 on Dextran Sodium Sulfate-Induced Colitis in Mice", JOURNAL OF MICROBIOLOGY AND BIOTECHNOLOGY, vol. 28, no. 11, 28 November 2018 (2018-11-28), Korea, pages 1800 - 1805, XP055820182, ISSN: 1017-7825, Retrieved from the Internet <URL:https://www.koreascience.or.kr/article/JAKO201836263122267.page> DOI: 10.4014/jmb.1805.05072

## Description

### Technical Field

The present invention relates to the technical field of microbiology, and in particular to a composition comprising *Bifidobacterium lactis* BL-99 with deposit number CGMCC 15650 for use in suppression of intestinal inflammation.

### Background Art

With the impacts of environmental factors, diet, and lifestyle habits on the microenvironment of intestinal flora, the incidence of enteritis is also increasing year by year, and enteritis has become one of the major diseases affecting human health worldwide. A large number of studies have shown that oxidative damage to intestinal cells results in mucosal damage, infection of intestinal epithelial cells, and release of toxins and other harmful substances, causing damage and death of intestinal epithelial cells, increased permeability of intestinal epithelium, and pathogenic bacteria crossing the damaged intestinal mucosal barrier, which triggers a series of immune responses, resulting in production of a large number of cytokines by macrophages, over-stimulation of T cells, production of pro-inflammatory factors, and inflammation of epithelial cells.

Traditional treatments of enteritis can be categorized into modern medical treatment and Chinese herbal treatment. With the development of science and technology, regulating the balance of intestinal flora through external supplementation of probiotics has also become an important method to reduce intestinal inflammation. As an endogenous immune defense barrier in the intestinal tract, probiotics are able to antagonize pathogenic bacteria, safe, controllable, and effective, and have few side effects, making them an ideal treatment for enteritis. The mechanism of probiotic treatment of enteritis is not well elucidated yet, but is generally attributed to the fact that probiotics can inhibit colonization of pathogenic bacteria through competition for nutrients and co-receptors, or directly inhibit pathogenic bacteria by production of bacteriocins, or isolate pathogenic bacteria and their toxins by production of antitoxin proteases. Probiotics adjust imbalanced immune responses and suppress host mucosal damage by maintaining normal intestinal flora, strengthening the mucosal barrier effect, and inhibiting exposure of the immune system to inflammatory signals. In addition, the World Gastrointestinal Organization (WGO) Global Guidelines indicates the use of probiotic preparations as a medicament for inflammatory bowel diseases.

Chae Jung Min ET AL: "Effects of Orally-Administered Bifidobacterium animalis subsp. lactis Strain BB12 on Dextran Sodium Sulfate-Induced Colitis in Mice", Journal of Microbiology and Biotechnology, vol. 28, no. 11, 2018-11-28, pages 1800-1805 discloses the results of a study of the effects of orally-administered Bifidobacterium animalis subsp. lactis strain BB 12 on dextran sodium sulfate-induced colitis in mice. It was found that oral administration of BB 12 markedly ameliorated DSS-induced colitis, accompanied by reduced tumor necrosis factor-a-mediated IEC apoptosis. These findings indicate that the probiotic strain BB12 can alleviate DSS-induced colitis and suggest a novel mechanism of communication between probiotic microorganisms and intestinal epithelia, which increases intestinal cell survival by modulating pro-apoptotic cytokine expression.

WO 2010/099824 A1 discloses a bacterium strain selected from the group consisting of L. paracasei LMG P-2138G, L, plantarum LMG P-21021, Bifidobacterium lactis LMG P-21384, and Bifidobacterium breve DSM 166C4 or its cellular components, as inducer of Interleukin-10. Also disclosed is a composition comprising said strain, for use as a medicament for the prevention or treatment of inflammatory conditions of the large intestine and small intestine, preferably selected from the group comprising Crohn's disease and ulcerative colitis. Summary of the Invention

The claimed invention is set out in the appended claims.

An objective of the present invention is to provide a new use of *Bifidobacterium lactis* BL-99.

The present invention provides a *Bifidobacterium lactis* strain, named BL-99 in the present invention. The strain has been deposited in the China General Microbiological Culture Collection Center CGMCC (Address: No. 3, Yard 1, Beichen West Road, Chaoyang District, Beijing, China, Institute of Microbiology, Chinese Academy of Sciences) on April 26, 2018, under the taxonomic designation *Bifidobacterium lactis*; deposit number: CGMCC 15650. The present invention provides *Bifidobacterium lactis* which is resistant to gastric acid and intestinal fluid, showing a survival rate of more than 62% after 30-minute treatment and 61% after 2-hour treatment in gastric acid at pH 2.5, and more than 70% after 2-hour treatment in small intestinal fluid at pH 6.8.

It was discovered in the studies of the present invention that *Bifidobacterium lactis* BL-99 (i.e., *Bifidobacterium lactis* with deposit number CGMCC 15650) alone was efficacious at inhibiting intestinal inflammation, reducing inflammatory factors IL-6 and/or TNF-α, promoting the anti-inflammatory factor IL-10, and reducing tissue damage in colitis.

The present invention provides *Bifidobacterium lactis* for use in suppressing intestinal inflammation, wherein the *Bifidobacterium lactis* has the deposit number CGMCC 15650. The *Bifidobacterium lactis* may be present in a form of a composition comprising it.

According to a specific embodiment of the present invention, the *Bifidobacterium lactis* is used in a form of solid or liquid bacterial preparation to manufacture the composition.

According to a specific embodiment of the present invention, the *Bifidobacterium lactis* is used in a form of viable and/or dead bacteria to manufacture the composition.

According to a specific embodiment of the present invention, the composition may comprise a food composition, a feed composition or a pharmaceutical composition.

According to a specific embodiment of the present invention, the composition may be administered to animals or humans. The composition may also comprise conventional ingredients in the art. For example, for a pharmaceutical composition, a suitable amount of auxiliary may be included, and the auxiliary may be an excipient, a diluent, a filler, an absorption enhancer, and the like. For a food composition, lactobifidobacteria according to the present invention can be produced according to food products containing lactobifidobacteria in the prior art, and the composition can be in different forms depending on the needs of the subject. Examples include powder, ingots, granulation, microcapsules, liquid formulations, and the like.

According to a specific embodiment of the present invention, the composition is for use in reducing inflammatory factors IL-6 and/or TNF-α. For specific applications, the *Bifidobacterium lactis* is used in an amount of 3.88×10⁶ CFU~3.88×10¹³ CFU/day, or 0.01 µg~100 mg/day by weight of the bacterium. Preferably, the *Bifidobacterium lactis* is used in an amount of 3.88×10⁸ CFU~3.88×10¹² CFU/day, or 0.1 µg~10 mg /day by weight of the bacterium.

According to a specific embodiment of the present invention, the composition is for use in promoting anti-inflammatory factor IL-10. For specific applications, the *Bifidobacterium lactis* is used in an amount of 3.88×10⁶ CFU~3.88×10¹³ CFU/day, or 0.01 µg~100 mg/day by weight of the bacterium. Preferably, the *Bifidobacterium lactis* is used in an amount of 3.88×10⁸ CFU~3.88×10¹² CFU/day, or 0.1 µg~10 mg /day by weight of the bacterium.

According to a specific embodiment of the present invention, the composition is for use in reducing tissue damage in colitis. For specific applications, the *Bifidobacterium lactis* is used in an amount of 3.88×10⁶ CFU~3.88×10¹³ CFU/day, or 0.01 µg~100 mg/day by weight of the bacterium. Preferably, the *Bifidobacterium lactis* is used in an amount of 3.88×10⁸ CFU~3.88×10¹² CFU/day, or 0.1 µg~10 mg /day by weight of the bacterium.

In a specific embodiment of the present invention, in addition to *Bifidobacterium lactis* BL-99, the composition may also comprise a biocompatible excipient to prepare a dosage form such as a solution, suspension, emulsion, powder, lozenge, pill, syrup, oral lozenge, tablet, chewing gum, or capsule, for general applications or pharmaceutical use.

In a specific embodiment of the present invention, the composition is a food composition, and the food may be a fermented dairy product (e.g. fermented milk, flavored fermented milk, a fermented milk beverage, and the like), cheese, a dairy-containing beverage, a solid beverage, dairy powder, or the like.

In another specific embodiment of the present invention, the composition is a feed composition. The other components in the feed composition can be selected with reference to conventional techniques in the field of probiotic feed.

In another specific embodiment of the present invention, the composition is a pharmaceutical composition. The other components in the pharmaceutical composition can be selected with reference to conventional techniques in the field of probiotic drugs.

### Brief Description of the Drawings

Fig. 1 shows the effect of *Bifidobacterium lactis* BL-99 on colonic IL-6 in mice.
Fig. 2 shows the effect of *Bifidobacterium lactis* BL-99 on colonic IL-10 in mice.
Fig. 3 shows the effect of *Bifidobacterium lactis* BL-99 on colonic TNF-α in mice.
Fig. 4 shows the results of pathological sections for the effect of *Bifidobacterium lactis* BL-99 on mice.
Fig. 5 shows the histology injury scores of mice under *Bifidobacterium lactis* BL-99.

### Microbe deposit for patent procedure:

*Bifidobacterium lactis* BL-99 of the present invention.
Date of deposit: 26/04/2018;
Depository Authority: China General Microbiological Culture Collection Center (CGMCC);
Address: No. 3, Yard 1, Beichen West Road, Chaoyang District, Beijing, China, Institute of Microbiology, Chinese Academy of Sciences;
Deposit number: CGMCC 15650;
Taxonomic designation: *Bifidobacterium lactis.*

### Detailed Description of the Invention

In order to provide a better understanding of the technical features, purpose and beneficial effects of the present invention, the following detailed description of the technical solutions of the present invention is provided in conjunction with specific examples, and it should be understood that these examples are used only to illustrate the invention and not to limit the scope of the invention.

In the examples, each original reagent and material are commercially available, and the experimental methods without specific conditions indicated are conventional methods under conventional conditions known in the art, or conducted under the conditions recommended by the manufacturer of instrument.

### Example 1: Bifidobacterium lactis BL-99 and its performance

*Bifidobacterium lactis* BL-99 of the present invention, from Shanghai Jiao Tong University Onlly Co., Ltd, was isolated from the intestine of infants. This strain has been deposited in the China General Microbiological Culture Collection Center CGMCC (Address: No. 3, Yard 1, Beichen West Road, Chaoyang District, Beijing, China, Institute of Microbiology, Chinese Academy of Sciences) on April 26, 2018 under the taxonomic designation *Bifidobacterium lactis*; with deposit number CGMCC 15650.

### 1. Taxonomic characteristics of Bifidobacterium lactis BL-99

### Physical and chemical test results:

| Test items | Results | | Test items | Results |
|---|---|---|---|---|
| Gram staining | positive | | Acid production from carbohydrates (continued) | |
| Cell shape | Rod-shaped, polymorphic | | Ribose | + |
| Formation of spores | - | | Trehalose | - |
| Contact enzyme assay | - | | Xylose | + |
| Oxidase | - | | Maltose | + |
| Growth in air | - | | Lactose | + |
| Anaerobic growth | + | | Raffinose | + |
| Acid production from carbohydrates | | | Sorbitol | - |
| | Mannose | - | Melibiose | + |
| | Melezitose | - | Galactose | + |
| | Fructose | - | Mannitol | - |
| | Salicin | + | L-Arabinose | - |
| | Synanthrin | - | Sodium gluconate | - |
| | Cellobiose | - | Saccharose | + |
| | Starch | + | | |

16S rRNA gene sequence (SEQ ID No. 1):

GCTCCCCCACAAGGGTCGGGCCACCGGCTTCGGGTGCTACCCACTTTCATGACTTGACGGG
CGGTGTGTACAAGGCCCGGGAACGCATTCACCGCGGCGTTGCTGATCCGCGATTACTAGCG
ACTCCGCCTTCACGCAGTCGAGTTGCAGACTGCGATCCGAACTGAGACCGGTTTTCAGCGA
TCCGCCCCACGTCACCGTGTCGCACCGCGTTGTACCGGCCATTGTAGCATGCGTGAAGCCC
TGGACGTAAGGGGCATGATGATCTGACGTCATCCCCACCTTCCTCCGAGTTGACCCCGGCG
GTCCCACATGAGTTCCCGGCATCACCCGCTGGCAACATGCGGCGAGGGTTGCGCTCGTTGC
GGGACTTAACCCAACATCTCACGACACGAGCTGACGACGACCATGCACCACCTGTGAACCG
GCCCCGAAGGAAACCGTGTCTCCACGGCGATCCGGCACATGTCAAGCCCAGGTAAGGTTC
TTCGCGTTGCATCGAATTAATCCGCATGCTCCGCCGCTTGTGCGGGCCCCCGTCAATTTCT
TTGAGTTTTAGCCTTGCGGCCGTACTCCCCAGGCGGGATGCTTAACGCGTTGGCTCCGACA
CGGGACCCGTGGAAAGGCCCCACATCCAGCATCCACCGTTTACGGCGTGGACTACCAGGG
TATCTAATCCTGTTCGCTCCCCACGCTTTCGCTCCTCAGCGTCAGTGACGGCCCAGAGACC
TGCCTTCGCCATTGGTGTTCTTCCCGATATCTACACATTCCACCGTTACACCGGAATTCC
AGTCTCCCCTACCGCACTCCAGCCCGCCCGTACCCGGCGCAGATCCACCGTTAGGCGATGG
ACTTTCACACCGGACGCGACGAACCGCCTACGAGCCCTTTACGCCCAATAAATCCGGATAA
CGCTCGCACCCTACGTATTACCGCGGCTGCTGGCACGTAGTTAGCCGGTGCTTATTCGAAC
AATCCACTCAACACGGCCGAAACCGTGCCTTGCCCTTGAACAAAGCGGTTTACAACCCGA
AGGCCTCCATCCCGCACGCGGCGTCGCTGCATCAGGCTTGCGCCCATTGTGCAATATTCCC
CACTGCTGCCTCCCGTAGGAGTCTGGGCCGTATCTCAGTCCCAATGTGGCCGGTCACCCTC
TCAGGCCGGCTACCCGTCAACGCCTTGGTGGGCCATCACCCCGCCAACAAGCTGATAGGAC
GCGACCCCATCCCATGCCGCAAAAGCATTTCCCACCCCACCATGCGATGGAGCGGAGCATC
CGGTATTACCACCCGTTTCCAGGAGCTATTCCGGTGCACAGGGCAGGTTGGTCACGCATTA
CTCACCCGTTCGCCACTCTCACCCCGACAGCAAGCTGCCAGGGATCCCGTTCGACT

### 2. Bifidobacterium lactis BL-99's tolerance towards artificial gastric and intestinal fluids

*Bifidobacterium* is a bacterial genus that is normally not resistant to acids. In this example, *Bifidobacterium lactis* BL-99's tolerance towards artificial gastric and intestinal fluids was tested, with *Bifidobacterium lactis* BB-12^{®} used as a reference, which is currently well known in the art as a strain having excellent acid resistance and capable of surviving the gastrointestinal tract.

Testing method: the *Bifidobacterium lactis* BL-99 strain was incubated in an MRS liquid medium at 37° C for 16 hours and then centrifuged at 4°C and 2500 rpm for 10 minutes to collect the bacterium.

The strains to be tested were cultured in an artificial gastric fluid and an artificial small intestine fluid, and the viable bacteria were counted and analyzed after treatment at 37°C for 0, 30 minutes and 2 hours, and the survival rate was used to evaluate the acid resistance and intestinal fluid resistance of the strains. Survival rate = (number of viable bacteria after treatment/number of viable bacteria at time 0) × 100%.

The results of the survival assay of the strains in the artificial gastric acid (pH 2.5) are shown in Table 1. The survival rate of BB-12 was 7.04% after 30-minute treatment in the artificial gastric acid (pH 2.5), and only 1.64% after 2-hour treatment, while the survival rate of *Bifidobacterium lactis* BL-99 according to the present invention was 62.60% after 30-minute treatment in the artificial gastric acid (pH 2.5), and 61.83% after 2-hour treatment, indicating that *Bifidobacterium lactis* BL-99 according to the present invention has excellent resistance to gastric acid and can pass stomach smoothly and reach intestine to exert probiotic effects.

**Table 1 Survival rates of strains in artificial gastric acid (pH 2.5)**

| Strains | Log CFU/ml (Survival rate, %) | | |
|---|---|---|---|
| | 0 min | 30 minutes | 2 hours |
| BB-12 | 8.78 (100) | 7.63 (7.04) | 7 (1.64) |
| BL-99 | 9.42 (100) | 9.21 (62.60) | 9.21 (61.83) |

The results of the survival assay of the strains in an artificial small intestine fluid (pH 6.8) are shown in Table 2. The data showed that the survival rate of BB-12 was only 28.95% after 2-hour treatment in the artificial small intestine fluid (pH 6.8), while the survival rate of *Bifidobacterium lactis* BL-99 according to the present invention was 70.23% after 2-hour treatment in the artificial small intestine fluid (pH 6.8), indicating that *Bifidobacterium lactis* BL-99 according to the present invention has excellent resistance to intestinal fluids and can survive and colonize the intestinal tract.

**Table 2 Survival rates of strains in artificial small intestine fluid (pH 6.8)**

| Strains | Log CFU/ml (Survival rate, %) | |
|---|---|---|
| | 0 min | 2 hours |
| BB-12 | 8.78 (100) | 8.24 (28.95) |
| BL-99 | 9.42 (100) | 9.26 (70.23) |

### 3. Toxicity test and safety test of Bifidobacterium lactis BL-99

*Bifidobacterium lactis* BL-99 according to the present invention was inoculated in a BBL liquid medium and incubated anaerobically at 36±1°C for 48±2 hours, and the viable cell count of *Bifidobacterium lactis* BL-99 in the culture was 3.7×10⁸ cfu/mL. The culture liquid as it was and a 5-fold concentrate of the culture liquid were given to the test mice by gavage at 20.0 mL/kg BW via mouth for 3 consecutive days, and the mice were observed for 7 days. The experiment was set up with the culture liquid as it was and a 5-fold concentrate thereof for comparison. The test results showed that the effects of the BBL culture liquid of *Bifidobacterium lactis* BL-99 and the 5-fold concentrate group on the body weight gain of the mice were not statistically significant (p > 0.05) as compared to their respective control groups, and no toxic responses or deaths were observed in the test mice.

The antibiotic susceptibility of *Bifidobacterium lactis* BL-99 was assessed using the method SN/T 1944-2007 "Determination of bacterial resistance in animals and preparations thereof". The evaluation results showed that *Bifidobacterium lactis* BL-99 was sensitive to Ampicillin, Penicillin G, Erythromycin, Chloramphenicol, Clindamycin, Vancomycin, and Tetracycline. This meets the requirements of the European Food Safety Authority (EFSA) for the drug resistance of bacteria in food. *Bifidobacterium lactis* BL-99 does not contain exogenous antibiotic resistance genes and is safe for consumption.

### Example 2: Experiment on the efficacy of Bifidobacterium lactis BL-99 at suppressing intestinal inflammation

### 1. Experimental materials

Healthy BABL/c male mice, purchased from Beijing Huafukang Biotechnology Co. Ltd., were bred in the animal house of CDC maintained at room temperature (25±2°C) and relative humidity of (55±2)%, under 12h/12h alternating day/night light, and allowed free access to food and water.

### 2. Experimental methods

### 2.1 Animal grouping and handling

112 healthy BABL/c male mice, aged 6-8 weeks and weighing 20-22 g, were randomly divided based on body weight into 8 groups, with 14 mice per group. Each group was bred in two cages with 7 animals per cage, numbered with picric acid, and adapted for 5 days with normal feed. The details of the groups and sample volumes are shown in Table 3. The mice were subjected to intervention by gavage with a volume of 0.4 ml/20 g. The intervention period was 14 days.

**Table 3 Experimental grouping**

| **Grouping** | **Test drug** | **Number of animals** | **Gavage volume for mice (cfu/20g)** |
|---|---|---|---|
| Control group | PBS | 14 | - |
| Model group | PBS | 14 | - |
| Low dose group | BL-99 | 14 | 1×10⁷ |
| Medium dose group | BL-99 | 14 | 1×10⁸ |
| High dose group | BL-99 | 14 | 1×10⁹ |
| Dead bacteria low dose group | Inactivated BL-99 | 14 | 1×10⁷ |
| Dead bacteria medium dose group | Inactivated BL-99 | 14 | 1×10⁸ |
| Dead bacteria high dose group | Inactivated BL-99 | 14 | 1×10⁹ |

The dead bacteria samples were samples of inactivated BL-99, i.e. BL-99 samples prepared according to requirements were inactivated by heating at 100°C for 20 minutes, metered to a volume in PBS, and refrigerated.

Among the eight groups of mice, except for the control group, 7 groups required DSS induction for establishment of an experimental colitis model. On day 8 of the experiment, a 5.0% aqueous solution of DSS was prepared to replace drinking water and the mice consumed it freely for 7 days, while the normal group consumed distilled water. The mice were observed every day for changes in physical signs.

### 2.2 Colon length and weight measurement

After the intervention, the mice were anesthetized by intraperitoneal injection of sodium pentobarbital, blood was taken from the abdominal aorta, and serum was separated by centrifugation. The colon of each mouse was isolated, rinsed several times with PBS, and measured for length, and 2/3 of the colon was cut and stored in a centrifuge tube at -80°C. The other 1/3 was stored in a 10% formalin solution for fixation.

### 2.3 Observation and scoring of histopathology of the colon

After the colon was fixed in the formalin solution, it was sequentially dehydrated, waxed, embedded, sectioned, bathed and baked, dewaxed and rehydrated, HE stained, and finally microscopically observed for histomorphology.

Histological scoring was performed using the Fedorak histological scoring criteria. The histology injury scoring criteria are shown in Table 4.

**Table 4 Histology injury scoring criteria**

| **Score** | **Inflammation** | **Lesion depth** | **Recess damage** | **Extent of lesion** |
|---|---|---|---|---|
| 0 | none | none | none | / |
| 1 | Mild | Submucosa | Basal 1/3 recess, damaged | 1%-25% |
| 2 | Medium | Muscular layer | Basal 2/3 recess, damaged | 26%-50% |
| 3 | / | Serosa layer | Intact surface epithelium only | 51%-75% |
| 4 | / | / | All recess and epithelium damaged | 76%-100% |

### 2.4 Measurement of cytokines in serum

The levels of cytokines IL-6, IL-10, and TNF-α in the colons of mice were measured according to the ELISA kit instructions.

### 2.5 Statistical analysis methods

Experimental data were expressed as Mean±S.E.M. Data were processed using PRISM version 5.0 (GraphPad, San Diego, CA, USA). Differences between groups were evaluated using one-way ANOVA following Tukery's multiple comparison test. P < 0.05 indicates a statistically significant difference.

### 3. Experimental results and analysis

### 3.1 Changes in body weight of mice

The body weights of mice at 0, 7, and 14 days were measured and the results are shown in Table 5.

**Table 5 Changes in body weight of mice**

| Grouping | Day 0 | Day 7 | Day 14 |
|---|---|---|---|
| Control group | 20.92±0.56 | 22.25±0.88 | 22.68±1.02 |
| Model group | 21.36±0.99 | 23.18±1.21 | 19.19±2.28* |
| Low dose group | 21.39±0.98 | 22.88±1.55 | 19.89±2.42* |
| Medium dose group | 21.22±0.64 | 23.06±1.38 | 19.37±1.71* |
| High dose group | 21.01±0.79 | 22.17±0.93 | 19.13±1.78* |
| Dead bacteria low dose group | 20.76±0.96 | 22.66±1.34 | 20.43±2.59* |
| Dead bacteria medium dose group | 21.39±1.10 | 22.15±1.61 | 19.12±2.70* |
| Dead bacteria high dose group | 21.04±1.39 | 23.16±1.85 | 19.51±2.35* |

| | | | |
|---|---|---|---|
| Note: *The difference is significant as compared with the control group. | | | |

On day 0, there was no significant difference (*p* < 0.05) in body weight between the mice groups, indicating that the mice were in the same condition at the beginning of the experiment, and the experimental deviation caused by a difference in body weight of the mice can be excluded. After 7 days of sample administration, the body weight of mice increased in all groups, with no significant difference (*p* < 0.05) in body weight between the mice groups, indicating that the short-time sample intervention had no effect on the body weight gain of mice. After 7 days under 5% DSS instead of drinking water, the body weight of all mice in the model group decreased significantly (*p* < 0.05), while there was no significant change in the body weight of the control group (*p* > 0.05). Meanwhile, the mouse status observation results indicated successful modeling in the model group. After modeling, the body weight of mice in both the model group and the intervention groups significantly decreased, indicating that despite the sample intervention, the body weight of mice still decreased due to the intestinal damage caused by DSS. After modeling, the body weight of mice in each intervention group was significantly lower than that in the control group (*p <* 0.05), but showed no significant difference from that in the model group (*p* > 0.05), indicating that the samples had a limited intervention effect on the body weight of mice molded by DSS.

### 3.2 Characterization of DSS-induced colonic inflammation in mice

From day 0 to day 7, mice in each group showed smooth fur, an active spirit, a quick response, normal feeding activity, and spherical or striped stools without diarrhea or bloody stools. After 7 days of modeling, mice in both the model group and the intervention groups were induced with 5.0% DSS to build an experimental colitis model. The changes in the physical signs of the mice in each experimental group during the modeling period were observed separately, and the relevant results are shown in Table 6.

**Table 6 Observation of physical signs of mice**

| Grouping | Number of mice | Time of diarrhea (n days after modeling) | Time of bloody stools (n days after modeling) | Number of mice with bloody stools | Number of deaths |
|---|---|---|---|---|---|
| Control group | 14 | - | - | 0 | 0 |
| Model group | 14 | 3 | 3 | 14 | 0 |
| Low dose group | 14 | 3 | 5 | 6 | 0 |
| Medium dose group | 14 | 3 | 5 | 7 | 2 |
| High dose group | 14 | 3 | 5 | 7 | 0 |
| Dead bacteria low dose group | 14 | 3 | 4 | 8 | 0 |
| Dead bacteria medium dose group | 14 | 3 | 3 | 9 | 0 |
| Dead bacteria high dose group | 14 | 3 | 3 | 8 | 0 |

The observation of intestinal inflammation symptoms in mice showed that the intervention effect of each sample on DSS-modeled mice was manifested in two aspects: (1) the number of mice with bloody stools decreased at the end of the experiment; (2) the time of appearance of bloody stools in mice was delayed by 1~2 days compared with the model group. Because this modeling was done with 5% DSS instead of drinking water, and probably because the mice had different uptake and tolerance of DSS, the number of death did not change with the dose.

### 3.3 Spleen weight of mice in the groups

The spleen weights of mice in each group are shown in Table 7. As compared with the control group, the splenic indices of mice in the model group were all significantly higher than those in the control group (*p* < 0.05), indicating that 5% DSS can stimulate proliferation of lymphocytes and macrophages in the spleen of mice and stimulate the body to exert cellular and humoral immunological functions. The BL-99-medium-dose group and the dead bacteria low-dose group showed a decreasing tendency in splenic index, suggesting that the BL99-medium-dose group and the inactivated bacteria low-dose group may have a function of reducing inflammatory responses of the organism.

**Table 7 The spleen weight and splenic index of mice**

| Grouping | Spleen weight (g) | Spleen index |
|---|---|---|
| Control group | 0.081±0.011 | 0.36±0.05 |
| Model group | 0.085±0.021 | 0.44±0.09* |
| Low dose group | 0.082±0.015 | 0.41±0.06 |
| Medium dose group | 0.075±0.012 | 0.39±0.06 |
| High dose group | 0.086±0.015 | 0.46±0.12* |
| Dead bacteria low dose group | 0.080±0.015 | 0.39±0.08 |
| Dead bacteria medium dose group | 0.085±0.019 | 0.44±0.09* |
| Dead bacteria high dose group | 0.086±0.011 | 0.45±0.06* |

| | | |
|---|---|---|
| Note: *The difference is significant as compared with the control group. | | |

### 3.4 Test indicators

### 3.4.1 Measurement of mouse colon length

The results of mouse colon length are shown in Table 8. After modelling, the colon length of mice in the model group was significantly lower than that of the control group (*p* < 0.05). After the sample intervention, there was no significant difference in colon length between each mice group and the model group (*p* > 0.05), indicating that the main effect on the colon length of mice in this experiment was from the 5% DSS, and the short-term sample intervention showed no significant effect on the colon length of mice.

**Table 8 Results of colon length measurement in mice**

| Grouping | Colon length (cm) |
|---|---|
| Control group | 12.51±0.92 |
| Model group | 7.78±1.53* |
| Low dose group | 7.81±1.10* |
| Medium dose group | 8.81±0.83* |
| High dose group | 8.15±1.47* |
| Dead bacteria low dose group | 8.40±1.09* |
| Dead bacteria medium dose group | 7.96±1.69* |
| Dead bacteria high dose group | 7.64±1.09* |

| | |
|---|---|
| Note: *The difference is significant as compared with the control group. | |

### 3.4.2 Colonic IL-6 assay results

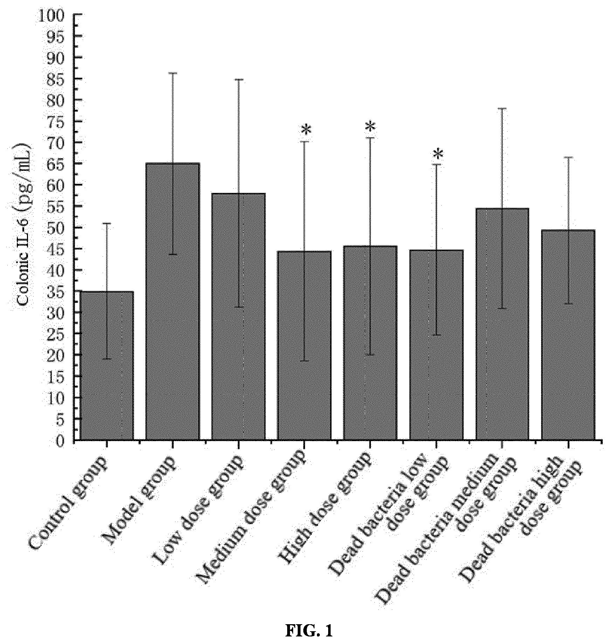

The results of changes in colonic IL-6 are shown in Fig. 1. As compared with the control group, colonic IL-6 was significantly higher in the model group mice (*p* < 0.05), indicating that DSS intervention in mice can cause an increase in intestinal inflammatory responses in mice, as evidenced by an increase in the inflammatory factor IL-6. As compared with the model group, the colonic IL-6 of mice in the BL-99-medium-dose group and the dead bacteria low-dose group was significantly lower than that in the model group (p < 0.05); indicating that the probiotic intervention at medium and high doses of BL-99 and a low dose of dead bacteria reduced intestinal inflammatory responses of mice.

### 3.4.3 Colonic IL-10 assay results

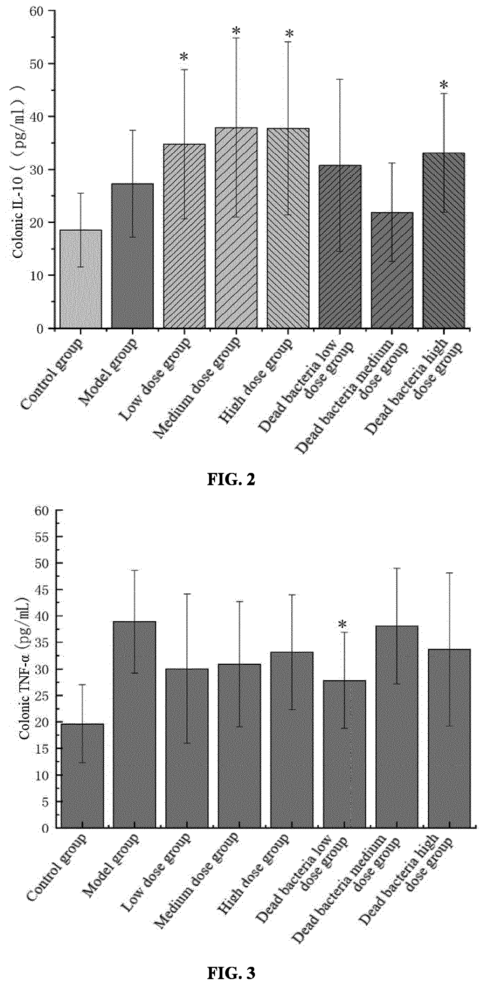

The results of changes in colonic IL-10 are shown in Fig. 2. As compared with the control group, colonic IL-10 in the model group increased but the difference was not significant (*p* > 0.05), indicating that the modelling has a tendency of causing increased secretion of the intestinal anti-inflammatory factor IL-10. As compared with the model group, colonic IL-10 in mice significantly increased in both the medium- and high-dose groups (p < 0.05), indicating that the probiotic intervention at medium and high doses of BL-99 had an effect of promoting production of the anti-inflammatory factor IL-10 by intestinal anti-inflammatory cells.

### 3.4.4 Colonic TNF-α assay results

The results of changes in colonic TNF-α are shown in Fig. 3. As compared with the model group, the low-, medium-, and high-dose groups of BL-99 and the dead bacteria high-dose group showed a decreasing trend for colonic TNF-α, with a significant decrease in mouse colonic TNF-α in the dead bacteria low-dose group (p<0.05), indicating that low and medium doses of dead probiotics can reduce intestinal inflammatory responses and reduce secretion of the colonic inflammatory factor TNF-α.

### 3.4.5 Pathology results

### 3.4.5.1 Pathological sections

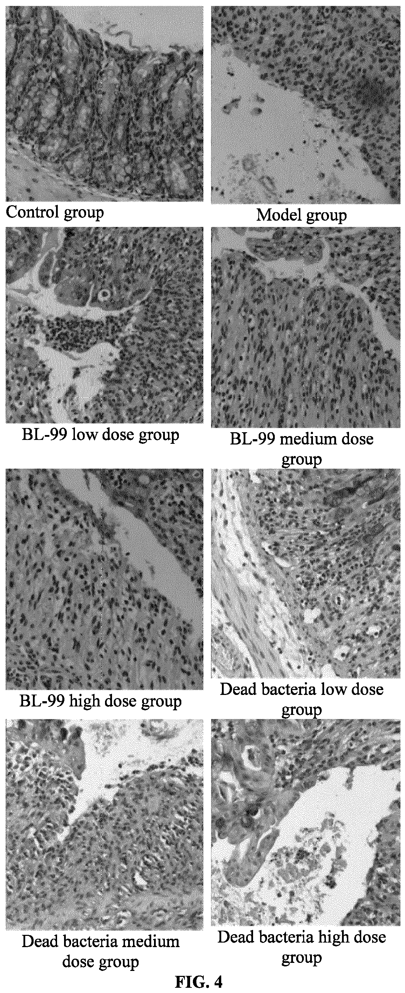

The results of pathological sections are shown in Fig. 4. Histological observation of mice in the control group showed intact colonic epithelial cells and clear recess structures and goblet cells. Histological observation of colitis mice in the model group induced by DSS showed that intact colonic epithelial cells could not be seen, and also showed incomplete recess and damaged goblet cells, with the damaged area being more than 50%, and in some mice the recesses disappeared completely and the goblet cells were destroyed completely. Inflammatory cell infiltration, such as neutrophils and lymphocytes, can also be observed in the mice.

Mice modeled by DSS after BL-99 intervention showed inflammatory cell infiltration, disappearance of a few recesses, and destruction of goblet cells, with a more severe inflammatory response in the medium-dose group, with lesions ranging from 50 to 75%, and more limited lesions in the low and high-dose groups, mostly in 0 to 25%. Mice modeled by DSS after dead bacteria intervention showed inflammatory cell infiltration, disappearance of recesses in a large area, and destruction of goblet cells, with more severe lesions, mostly in the 50% range.

### 3.4.5.2 Analysis of histology injury scores in mice

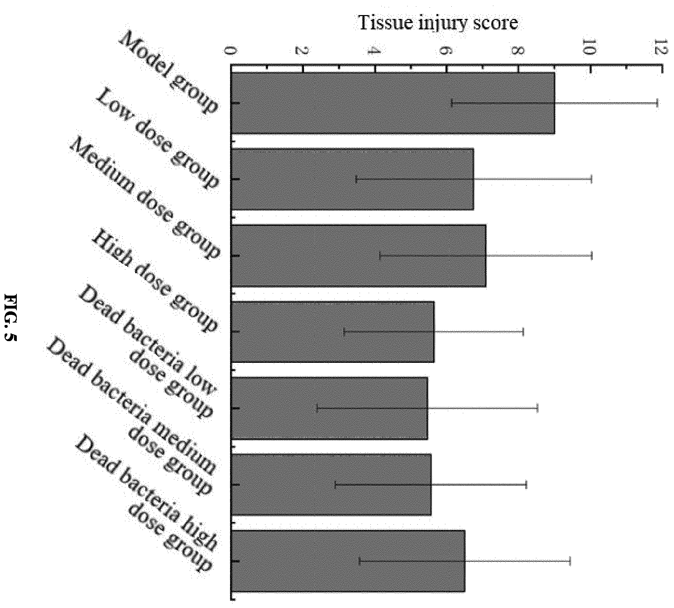

The histology injury scores are shown in Fig. 5. As compared with the model group, the histological injury scores of all groups showed a decreasing trend, among which the histology injury scores of the BL-99 low- and high-dose groups and the dead bacteria low-, medium-, and high-dose groups were significantly lower than that of the model group (*p <* 0.05), indicating that the probiotics in the above groups had an effect of reducing the symptoms of colonic inflammation in mice.

DSS-induced colitis is the most common method for establishing experimental animal colitis models, which are usually formed by allowing mice to drink freely for about 7 days. In this test, 5% DSS was used as the modeling concentration, and the model group had no death, and the mice started to have bloody stools on the third day, with the blood in stools and the number of mice having bloody stools increasing with time, indicating that the model was established with good stability.

The mice in each BL-99 dose group showed bloody stools later than the model group, had the number of mice having bloody stools less than that of the model group, and showed milder symptoms, which directly indicates the anti-inflammatory effect of the probiotic. In contrast, the mice in the dead bacteria intervention groups had the same time of showing bloody stools as the model group, and the bloody stools appeared more frequently because the inactivated probiotic bacteria have a reduced regulatory effect on the intestine, which led to a higher incidence of intestinal inflammation.

IL-6 is a multifunctional crucial cytokine that regulates expression of other cytokines. In the course of DSS-induced experimental colitis, the level of expression of IL-6, a pro-inflammatory factor, was closely related to the degree of inflammation in colitis, and mice lacking IL-10 exhibited severe intestinal inflammation, and IL-10 showed good therapeutic effects in animal models of colitis. TNF-α is a cytokine involved in systemic inflammation.

In this study, colonic IL-6 was significantly elevated in the mice in the model group, while colonic IL-6 in the mice in the BL-99 medium- and high-dose groups and the dead bacteria low-dose group was lower than that in the model group, and the level of colonic anti-inflammatory factor IL-10 was increased in the mice in the BL-99 medium- and high-dose groups, indicating that the medium- and high-doses of BL-99 promoted IL-10 in colitis and enhanced anti-inflammatory effects, suggesting that both BL-99 and inactivated probiotics can reduce intestinal inflammatory responses in mice and alleviate the symptoms of DSS-induced experimental colitis.

The mice after the BL-99 intervention and the inactivated bacteria intervention showed significant differences in colonic tissue injury score from the model group, as evidenced by the lower degree of inflammatory cell infiltration, lesion depth mainly in the submucosa, less destruction of the basal recesses, and smaller lesion extent in the sections, which visually showed that BL-99 viable and dead cells can reduce the degree of colonic inflammation in the DSS-modeled mice.

The above results confirmed that *Bifidobacterium lactis* BL99 significantly inhibits inflammatory factors IL-6 and TNF-α, elevates the level of anti-inflammatory factor IL-10, recovers the loss of colonic tissue, and is useful in food products such as fermented milk, cheese, milk-containing beverages, milk powder or any other kind of food containing the strain or derivatives thereof.

## Claims

1. A composition comprising *Bifidobacterium lactis* for use in suppressing intestinal inflammation, wherein the *Bifidobacterium lactis* has the deposit number CGMCC 15650.

2. The composition for use according to claim 1, wherein the *Bifidobacterium lactis* is in a form of solid or liquid bacterial preparation of viable and/or dead bacteria in the composition.

3. The composition for use according to claim 1, wherein the composition comprises a food composition, a feed composition, or a pharmaceutical composition.

4. The composition for use according to claim 1, wherein the composition is for use in reducing inflammatory factors IL-6 and/or TNF-α.

5. The composition for use according to claim 4, wherein the *Bifidobacterium lactis* is used in an amount of 3.88×10⁶ CFU to 3.88×10¹³ CFU/day or 0.01 µg to 100 mg/day.

6. The composition for use according to claim 1, wherein the composition is for use in promoting anti-inflammatory factor IL-10.

7. The composition for use according to claim 6, wherein the *Bifidobacterium lactis* is used in an amount of 3.88×10⁶ CFU to 3.88×10¹³ CFU/day or 0.01 µg to 100 mg/day.

8. The composition for use according to claim 1, wherein the composition is for use in reducing tissue damage in colitis.

9. The composition for use according to claim 8, wherein the *Bifidobacterium lactis* is used in an amount of 3.88×10⁶ CFU to 3.88×10¹³ CFU/day or 0.01 µg to 100 mg/day.

10. The composition for use according to any one of claims 4-9, wherein the composition is a food composition, preferably, the food is a fermented dairy product, cheese, a dairy-containing beverage, a solid beverage, or dairy powder.

## Patentansprüche

1. Zusammensetzung, umfassend *Bifidobacterium lactis,* zur Verwendung bei der Unterdrückung einer intestinalen Entzündung, wobei *Bifidobacterium lactis*die Hinterlegungsnummer CGMCC 15650 aufweist.

2. Zusammensetzung zur Verwendung nach Anspruch 1, wobei das *Bifidobacterium lactis* in Form eines festen oder flüssigen Bakterienpräparats von lebensfähigen und/oder toten Bakterien in der Zusammensetzung vorliegt.

3. Zusammensetzung zur Verwendung nach Anspruch 1, wobei die Zusammensetzung eine Nahrungsmittelzusammensetzung, eine Futtermittelzusammensetzung oder eine pharmazeutische Zusammensetzung umfasst.

4. Zusammensetzung zur Verwendung nach Anspruch 1, wobei die Zusammensetzung zur Verwendung bei der Reduzierung der Entzündungsfaktoren IL-6 und/oder TNF-α dient.

5. Zusammensetzung zur Verwendung nach Anspruch 4, wobei das *Bifidobacterium lactis* in einer Menge von 3,88×10⁶ KBE bis 3,88×10¹³ KBE/Tag oder 0,01 µg bis 100 mg/Tag verwendet wird.

6. Zusammensetzung zur Verwendung nach Anspruch 1, wobei die Zusammensetzung zur Förderung des entzündungshemmenden Faktors IL-10 verwendet wird.

7. Zusammensetzung zur Verwendung nach Anspruch 6, wobei das *Bifidobacterium lactis* in einer Menge von 3,88×10⁶ KBE bis 3,88×10¹³ KBE/Tag oder 0,01 µg bis 100 mg/Tag verwendet wird.

8. Zusammensetzung zur Verwendung nach Anspruch 1, wobei die Zusammensetzung zur Verwendung bei der Reduzierung von Gewebeschädigung bei Kolitis verwendet wird.

9. Zusammensetzung zur Verwendung nach Anspruch 8, wobei das *Bifidobacterium lactis* in einer Menge von 3,88×10⁶ KBE bis 3,88×10¹³ KBE/Tag oder 0,01 µg bis 100 mg/Tag verwendet wird.

10. Zusammensetzung zur Verwendung nach einem der Ansprüche 4-9, wobei die Zusammensetzung eine Nahrungsmittelzusammensetzung ist, bevorzugt das Nahrungsmittel ein fermentiertes Milchprodukt, Käse, ein Milchprodukt enthaltendes Getränk, ein festes Getränk oder Milchproduktpulver ist.

## Revendications

1. Composition comprenant du *Bifidobacterium lactis* destinée à être utilisée pour supprimer l'inflammation intestinale, dans laquelle le *Bifidobacterium lactis* présente le numéro de dépôt CGMCC 15650.

2. Composition destinée à être utilisée selon la revendication 1, dans laquelle le *Bifidobacterium lactis* se présente sous forme d'une préparation bactérienne solide ou liquide de bactéries viables et/ou mortes dans la composition.

3. Composition destinée à être utilisée selon la revendication 1, dans laquelle la composition comprend une composition alimentaire, une composition d'alimentation ou une composition pharmaceutique.

4. Composition destinée à être utilisée selon la revendication 1, dans laquelle la composition est destinée à être utilisée pour réduire les facteurs inflammatoires IL-6 et/ou TNF-α.

5. Composition destinée à être utilisée selon la revendication 4, dans laquelle le *Bifidobacterium lactis* est utilisé en une quantité de 3,88×10⁶ UFC à 3,88×10¹³ UFC/jour ou de 0,01 µg à 100 mg/jour.

6. Composition destinée à être utilisée selon la revendication 1, dans laquelle la composition est destinée à être utilisée pour favoriser le facteur anti-inflammatoire IL-10.

7. Composition destinée à être utilisée selon la revendication 6, dans laquelle le *Bifidobacterium lactis* est utilisé en une quantité de 3,88×10⁶ UFC à 3,88×10¹³ UFC/jour ou de 0,01 µg à 100 mg/jour.

8. Composition destinée à être utilisée selon la revendication 1, dans laquelle la composition est destinée à être utilisée pour réduire des lésions tissulaires dans la colite.

9. Composition destinée à être utilisée selon la revendication 8, dans laquelle le *Bifidobacterium lactis* est utilisé en une quantité de 3,88×10⁶ UFC à 3,88×10¹³ UFC/jour ou de 0,01 µg à 100 mg/jour.

10. Composition destinée à être utilisée selon l'une quelconque des revendications 4-9, dans laquelle la composition est une composition alimentaire, de préférence l'aliment est un produit laitier fermenté, du fromage, une boisson contenant un produit laitier, une boisson solide ou une poudre laitière.
